# EUROPEAN PATENT APPLICATION

(11) **EP 4 674 973 A1**
(43) Date of publication of application: **07.01.2026**
(21) Application number: 24763295.3
(22) Date of filing: 12.04.2024
(51) Int. Cl.: C12P 19/02, C12N 9/02

(54) **METHOD FOR PREPARING D-GLUCURONIC ACID AND PROCESS FOR PREPARING GLUCUROLACTONE**

(30) Priority: 28.02.2023 CN 202310177817; 22.03.2023 CN 202310283516
(71) Applicant: Zhucheng Howtian Pharm Co., Ltd, Weifang, Shandong 262218 (CN)
(72) Inventor: ZHU, Liping, Weifang, Shandong 262218 (CN); XU, Liangping, Weifang, Shandong 262218 (CN); HUAI, Jianlu, Weifang, Shandong 262218 (CN); QIU, Chongshun, Weifang, Shandong 262218 (CN); LIAO, Fei, Weifang, Shandong 262218 (CN); ZHENG, Binhua, Weifang, Shandong 262218 (CN); WEI, Pingping, Weifang, Shandong 262218 (CN)
(74) Representative: Zaboliene, Reda
(86) International application number: PCT/CN2024/087582
(87) International publication number: WO 2024/179615

(57) **Abstract**

A method for producing D-glucuronic acid by means of biological fermentation and a method for preparing glucurolactone using glucuronic acid, which belong to the technical field of biological preparation. The methods comprise the following steps: culturing recombinant engineering bacteria to obtain a seed solution; inoculating the seed solution onto a fermentation tank culture medium for fermentation culture; obtaining a fermentation solution by means of feeding control and induction control; performing centrifugation or membrane filtration on the fermentation solution to obtain a wet thallus; and adding the wet thallus to a reaction solution for conversion to obtain D-glucuronic acid. Furthermore, adding the D-glucuronic acid solution to a concentrated phosphoric acid, performing an esterification reaction under stirring conditions at a reaction temperature of 40-80°C, and crystallizig same to obtain a crude glucurolactone. By using the fermentation process to produce D-glucuronic acid, the inositol conversion rate can reach 95% or above, the amount of obtained D-glucuronic acid is higher than 76 g/L, and the inositol oxidase can also be reused. The time from the reaction to the completion of crystallization is less than 6 hours. Therefore, the reaction and crystallization time is greatly shortened, the production cycle is shortened, and the production efficiency is improved.

## Description

The present application claims priority to Chinese Patent Application No. 202310177817.5 filed to the China National Intellectual Property Administration (CNIPA) on February 28, 2023 and entitled "PREPARATION PROCESS OF GLUCURONOLACTONE" and Chinese Patent Application No. 202310283516.0 filed to the China National Intellectual Property Administration (CNIPA) on March 22, 2023 and entitled "METHOD FOR PRODUCING D-GLUCURONIC ACID (GlcUA) THROUGH BIOLOGICAL FERMENTATION", which are incorporated herein by reference in their entireties.

### TECHNICAL FIELD

The present disclosure relates to the technical field of biological preparation, and in particular to a method for producing D-glucuronic acid (GlcUA) through biological fermentation and a process for preparing glucuronolactone with GlcUA.

### BACKGROUND

Inositol, also known as cyclohexanehexol, hexahydroxycyclohexane, cyclohexanol, myo-inositol, and D-chiro-inositol, has a molecular formula of C₆H₁₂O₆ and a molecular weight of 180. Inositol is one of the B vitamins. Due to the different orientations of hydroxyl relative to a ring plane, there are 9 isomers of inositol, with 7 non-optical isomers and 2 optical isomers (a levoisomer and a dextroisomer). Inositol is widely distributed in animals and plants, and is a growth factor for animals and microorganisms. Inositol is mainly used for treating liver cirrhosis, hepatitis, fatty liver, high blood cholesterol, etc. Since inositol is an essential substance for the growth of humans, animals, and microorganisms, inositol is widely used in fields such as feed, medicine, and food.

GlcUA has a molecular formula of C₆H₁₀O₇ and a molecular weight of 194.14. As a biological detoxicant, GlcUA can bind to endogenous and exogenous toxic substances including groups such as hydroxyl, amino, carboxyl, and sulfhydryl in the animal liver to improve the water solubility of these toxic substances, such that these toxic substances can be finally excreted in the form of glucuronate esters, glucuronate salts, or complexes, thereby playing a detoxification role. In addition, it has been proved that GlcUA has the functions of preventing and treating the dermatitis and reducing the cholesterol and triglyceride concentrations in blood. Thus, GlcUA is also widely used in the beverage, food, and cosmetics industries. Glucuronolactone, commonly known as glucurone, is a derivative of GlcUA. Glucuronolactone can serve as a detoxicant and an immunomodulator in the liver. Glucuronolactone can be used for the treatment of liver diseases, the detoxification for foods and drugs, and the adjuvant therapy of rheumatoid arthritis. Due to the above applications, the annual demand for GlcUA increases progressively.

Traditionally, GlcUA is produced mainly by polysaccharide hydrolysis and chemical oxidation catalysis. The polysaccharide hydrolysis refers to a process of hydrolyzing a glycuronic acid-containing polysaccharide to produce GlcUA. For example, GlcUA is acquired from a water-soluble part of hemicellulose in a sunflower cell membrane. GlcUA is prepared through the alkaline hydrolysis of cotton and cellulose, the extraction of holocellulose with hot water, the oxidation of cellulose by an aqueous chlorine solution, etc. However, during the polysaccharide hydrolysis, because glycosidic bonds linking glycuronic acids generally have high stability and are difficult to hydrolyze, a hydrolysis process requires a strong acid or alkali. Under the strong acid or alkali, the product of GlcUA is often decomposed to cause defects such as poor oxidation selectivity, many by-products, and low product yield, which cannot meet the production requirements. The chemical oxidation catalysis refers to the oxidation of saccharides and derivatives thereof using an inorganic reagent to produce GlcUA. The nitric acid oxidation is the most widely used method to produce GlcUA. A process of the nitric acid oxidation is as follows: Starch is first oxidized with concentrated nitric acid to produce a crude oxidized starch solution. Then the crude oxidized starch solution is heated and pressurized under acidic conditions for hydrolysis to produce a hydrolysate. The hydrolysate is vacuum-concentrated, and acetic acid is added for esterification. Finally, the freeze crystallization is conducted to produce glucuronolactone. However, the nitric acid oxidation allows a relatively-low total yield (about 10%), and has disadvantages such as high energy consumption, poor selectivity, and serious environmental pollution. Therefore, the nitric acid oxidation still cannot meet the production requirements.

With the improvement of people's understanding for microorganisms, there are more and more studies on the biocatalytic production of GlcUA, myo-inositol oxygenase is an enzyme for promoting the conversion of inositol into GlcUA. However, the myo-inositol oxygenase has poor stability, which can easily lead to the incomplete conversion during a conversion process. As a result, when GlcUA is produced with the myo-inositol oxygenase, there are problems such as low product concentration and low conversion rate.

Glucuronolactone, a derivative of GlcUA, is prepared mainly by the following method: Starch is added to nitric acid with a content of about 80% (V/V) for oxidation to produce an oxidized starch solution. The oxidized starch solution is heated and pressurized under acidic conditions for hydrolysis to produce a hydrolysate with GlcUA as a main component. The hydrolysate is vacuum-concentrated to a Baume scale of 44 to 49, and then a compound acid reagent composed of phosphoric acid and sulfuric acid is added to allow a static esterification reaction at 10°C to 35°C for 24 h or more. After the static esterification reaction is completed, an alcohol is added to obtain a crystallization system, and the crystallization system is stirred and then cooled to 10°C to 15°C from 0 h to 24 h, cooled to 0°C or less from 24 h to 48 h, and then cooled to -8°C or less from 48 h to 60 h, so as to allow crystallization. In the above-mentioned technical solution, the total time of cooling crystallization is generally 70 h to 80 h, and there are problems such as cumbersome process steps, long production time, low efficiency, and low raw material utilization. Moreover, because the mixing of sulfuric acid and phosphoric acid will release a large amount of heat, the addition of both sulfuric acid and phosphoric acid causes some potential safety hazards and also increases the uncontrollable factors for a temperature of a reaction system. During a reaction process, a reaction temperature should be strictly controlled at 35°C or less, and glucuronolactone crystal grains produced accordingly are fine, resulting in large filtration difficulty.

### SUMMARY

In view of this, an objective of the present disclosure is to provide a preparation method of GlcUA and a method for preparing glucuronolactone with GlcUA. The present disclosure is intended to overcome the problem that the incomplete conversion occurs during a conversion process due to poor myo-inositol oxygenase stability to finally cause problems such as low product concentration and low conversion rate for the production of GlcUA with myo-inositol oxygenase in the prior art, and to overcome the problems such as cumbersome process steps, long production time, low efficiency, fine glucuronolactone crystal grains, and large filtration difficulty for the preparation of glucuronolactone in the prior art.

In order to achieve the above objective, in a first aspect, the present disclosure provides the following technical solutions: The present disclosure provides a preparation method of GlcUA, including the following steps:
(1) cultivating a recombinant engineered strain to produce a seed culture;
(2) inoculating the seed culture into a fermentation tank medium, and conducting fermentation cultivation to produce a fermentation broth;
(3) collecting a wet strain cell from the fermentation broth through centrifugation or membrane filtration; and
(4) adding the wet strain cell to a reaction solution for conversion to produce the GlcUA,
   where the step (2) includes: after the seed culture is inoculated into the fermentation tank medium for cultivation, conducting feeding control and induction control.

The method for producing GlcUA through biological fermentation provided by the present disclosure includes: cultivating a recombinant engineered strain to produce a seed culture; inoculating the seed culture into a fermentation tank medium, and conducting fermentation cultivation under feeding control and induction control to produce a fermentation broth; collecting a wet strain cell from the fermentation broth through centrifugation or membrane filtration; and adding the wet strain cell to a reaction solution for conversion to produce the GlcUA. In the method of the present disclosure, the excellent expression of the engineered strain is promoted by controlling the parameters of fermentation and conversion processes and especially by controlling compositions of media (a seed tank medium and the fermentation tank medium) and adopting the feeding control during fermentation to obtain high-activity, high-stability, and high-content myo-inositol oxygenase in the wet strain cell, which can promote the conversion rate of inositol in a conversion process and improve the concentration of GlcUA in a product. The preparation method of GlcUA in the present disclosure can improve a conversion rate of inositol to 95% or more, and leads to a product with a GlcUA content of 76 g/L, which reduces the extraction difficulty. In addition, the preparation method of GlcUA in the present disclosure allows the recycling of myo-inositol oxygenase and can reduce the production cost.

In a second aspect, the present disclosure provides a preparation process of glucuronolactone, including the following steps:
adding concentrated phosphoric acid to a GlcUA solution, conducting an esterification reaction at 40°C to 80°C under stirring, and allowing crystallization to produce a crude glucuronolactone product.

Compared with the prior art, in the preparation process of glucuronolactone in the present disclosure, the concentrated phosphoric acid is adopted instead of the mixed acid of phosphoric acid and sulfuric acid in the prior art, and a phosphoric acid concentration and a reaction temperature are controlled to allow the esterification reaction of the GlcUA solution under stirring to produce the glucuronolactone. When the technical solution of the present disclosure is used to prepare glucuronolactone, the total time from the starting of a reaction to the completion of crystallization is less than 6 h, which greatly shortens the reaction-crystallization time, reduces the production cycle, and improves the production efficiency. Moreover, in the esterification reaction system of the present disclosure, only concentrated phosphoric acid is adopted and a temperature is controllable, such that the prepared glucuronolactone has advantages such as large crystal grains, easy filtration, and high yield.

### BRIEF DESCRIPTION OF THE DRAWINGS

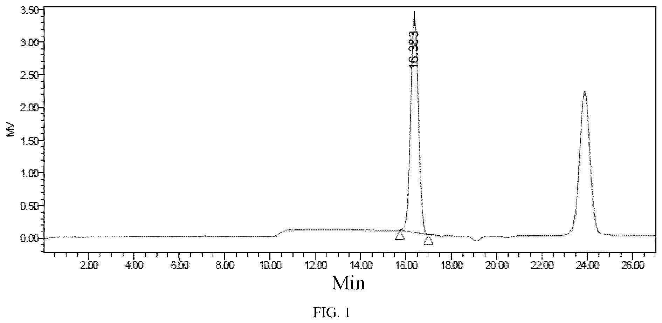

FIG. 1 shows a liquid chromatography spectrum of a glucuronolactone product prepared in Example 4 of the present disclosure.

### DETAILED DESCRIPTION OF THE EMBODIMENTS

To make the technical problems to be solved, the technical solutions, and the beneficial effects of the present disclosure clear, the present disclosure is described in further detail below with reference to specific embodiments. It should be understood that the specific embodiments described herein are merely intended to explain the present disclosure, rather than to limit the present disclosure.

The present disclosure provides a preparation method of GlcUA, including the following steps:
(1) A recombinant engineered strain is cultivated to produce a seed culture.
(2) The seed culture is inoculated into a fermentation tank medium, and fermentation cultivation is conducted to produce a fermentation broth.
(3) A wet strain cell is collected from the fermentation broth through centrifugation or membrane filtration.
(4) The wet strain cell is added to a reaction solution for conversion to produce the GlcUA.

In the present disclosure, a recombinant engineered strain is cultivated to produce a seed culture. In the present disclosure, the cultivation of the recombinant engineered strain includes the two steps of primary seed cultivation and secondary seed cultivation. The primary seed cultivation includes: the recombinant engineered strain is inoculated into an LB medium, and cultivated for 5 h to 6 h at 200 rpm to 240 rpm and 34°C to 40°C. The recombinant engineered strain in the present disclosure is purchased from the Institute of Microbiology, Chinese Academy of Sciences. The recombinant engineered strain has been patented and granted a patent with an application number CN201710790108.9. It should be understood that a substance actually catalyzing the conversion of inositol into GlcUA in the present disclosure is not the recombinant engineered strain itself, but is myo-inositol oxygenase produced through the functional protein expression in the recombinant engineered strain. The present disclosure has no special restrictions on the LB medium, and the LB medium is a medium well known in the art. Any commercially-available LB medium can be adopted in this solution. The present disclosure has no special restrictions on a container/dish for the primary seed cultivation, and a corresponding container or dish that can allow shaking cultivation can be adopted. Preferably, a shake flask can be adopted. The present disclosure has no special restrictions on a manner of shaking. Preferably, a shaker or an oscillator is adopted, and more preferably, a shaker is adopted. In the present disclosure, the shaking cultivation is conducted at a rate of preferably 200 rpm to 240 rpm and more preferably 220 rpm. In the present disclosure, the cultivation is conducted at preferably 34°C to 40°C and more preferably 37°C. In the present disclosure, the cultivation is conducted for preferably 5 h to 6 h and more preferably 5 h. It should be understood that the present disclosure adopts a combined cultivation mode of a shake flask + a shaker, which can increase an oxygen in a solution to meet the demand of blocking the cellular respiration.

Further, with reference to the above embodiment, after a primary seed culture is obtained, the present disclosure further includes: the primary seed culture is inoculated into a seed tank medium to allow secondary seed cultivation. The inoculation is conducted preferably when OD₆₀₀ is 2 to 3 and more preferably when OD₆₀₀ is 2.5. Conditions for the secondary seed cultivation are as follows: a temperature: preferably 34°C to 40°C and more preferably 37°C; an air flow rate: preferably 0.4 m³/h to 0.6 m³/h and more preferably 0.5 m³/h; a rotational speed: preferably 280 rpm to 320 rpm and more preferably 300 rpm; a pressure: preferably 0.01 MPa to 0.03 MPa and more preferably 0.02 MPa; a pH: adjusting with ammonia water to a pH of preferably 7.0±0.1 and more preferably 7.0; and a dissolved oxygen content: preferably 20% to 30% and more preferably 25%. An end point of the secondary seed cultivation in the present disclosure is determined according to an OD₆₀₀ value. The secondary seed cultivation is completed preferably when the OD₆₀₀ value reaches 2 to 3 and more preferably when the OD₆₀₀ value is 2. Specifically, in the present disclosure, after the secondary seed cultivation is conducted for 4 h, a sample is collected every hour and tested for OD₆₀₀ to monitor an OD₆₀₀ value of a system in real time. It should be understood that the secondary seed cultivation is a process of allowing the expansion and rapid growth of the strain to meet the needs of fermentation tank cultivation subsequently. Therefore, the selection of the above cultivation conditions is intended to enable the prominent expansion and growth of the strain.

Further, with reference to the above embodiment, in the present disclosure, a specific composition of the seed tank medium is preferably as follows: glucose: 1 wt% to 2 wt%, monopotassium phosphate: 1 wt% to 2 wt%, magnesium sulfate: 0.05 wt% to 0.08 wt%, citric acid: 0.1 wt% to 0.2 wt%, ammonium sulfate: 0.4 wt% to 0.6 wt%, trace elements: 1,000 mg/L to 1,500 mg/L, and a defoaming agent: 0.05 ml/L to 0.15 ml/L. In the present disclosure, the trace elements in the seed tank medium are preferably the following components: 20 mg/L to 30 mg/L of CoCl₂.6H₂O, 100 mg/L to 200 mg/L of MnSO₄.4H₂O, 10 mg/L to 20 mg/L of CuCl₂.2H₂O, 20 mg/L to 40 mg/L of H₃BO₃, 20 mg/L to 30 mg/L of Na₂MoO₄.2H₂O, 100 mg/L to 150 mg/L of Zn(CH₃COO)₂.2H₂O, and 500 mg/L to 1,500 mg/L of Fe(III) citrate. More preferably, the trace elements are the following components: 25 mg/L of CoCl₂.6H₂O, 150 mg/L of MnSO₄.4H₂O, 15 mg/L of CuCl₂.2H₂O, 30 mg/L of H₃BO₃, 25 mg/L of Na₂MoO₄.2H₂O, 130 mg/L of Zn(CH₃COO)₂.2H₂O, and 1,000 mg/L of Fe(III) citrate. It should be understood that the trace elements in the seed tank medium of the present disclosure play the following two roles: 1. The trace elements serve as components constituting cells of the strain. 2. The trace elements serve as components for active groups of the enzyme or can maintain an activity of the enzyme. The addition of the trace elements in the present disclosure greatly improves the activity and stability of myo-inositol oxygenase produced by the recombinant engineered strain, and provides a prominent foundation for the subsequent biological fermentation and conversion.

Further, with reference to the above embodiment, the present disclosure has no special restrictions on a type of the defoaming agent, and preferably, a commercially-available liquid defoaming agent is adopted. In the present disclosure, the defoaming agent is added at an amount of preferably 0.05 mL/L to 0.15 mL/L and more preferably 0.1 mL/L. It should be understood that the defoaming agent in the present disclosure can be added selectively according to a specific situation. For example, if there is too much foam on a liquid surface, the defoaming agent can be added manually according to a situation. The amount of the defoaming agent added should not be too large, and it is preferable to retain a small amount of foam. Otherwise, it is easy to cause the insufficient oxygen supply.

In the present disclosure, after the seed culture is obtained after the two stages of seed cultivation, the seed culture is inoculated into a fermentation tank medium, and fermentation cultivation is conducted to produce a fermentation broth. In the present disclosure, the fermentation cultivation is conducted under the following conditions: a temperature: preferably 34°C to 40°C and more preferably 37°C; an air flow rate: preferably 1.2 m³/h to 1.8 m³/h and more preferably 1.5 m³/h; a rotational speed: preferably 180 rpm to 220 rpm and more preferably 200 rpm; a pressure: preferably 0.01 MPa to 0.03 MPa and more preferably 0.02 MPa; a pH: adjusting with ammonia water to a pH of preferably 7.0±0.1 and more preferably 7.0; and a dissolved oxygen content: preferably 20% to 30% and more preferably 25%. In the present disclosure, a fermentation reaction is conducted for preferably 36 h to 40 h and more preferably 38 h. It should be understood that an indicator to measure the fermentation is generally as follows: when an OD₆₀₀ value remains stable, it indicates that the fermentation is completed or tends to be completed. The above 36 h to 40 h refers to a time of complete fermentation obtained according to the production experience. It should be understood that the fermentation cultivation is intended to allow the high expression of the strain on the basis of the above-mentioned seed cultivation. In the present disclosure, myo-inositol oxygenase is also produced for the subsequent inositol conversion process through the functional protein expression in the recombinant engineered strain.

Further, with reference to the above embodiment, in the present disclosure, a specific composition of the fermentation tank medium is preferably as follows: glucose: 1 wt% to 2 wt%, monopotassium phosphate: 1 wt% to 2 wt%, magnesium sulfate: 0.05 wt% to 0.08 wt%, citric acid: 0.1 wt% to 0.2 wt%, ammonium sulfate: 0.4 wt% to 0.6 wt%, trace elements: 1,000 mg/L to 1,500 mg/L, and a defoaming agent: 0.05 ml/L to 0.15 ml/L. In the present disclosure, the trace elements in the fermentation tank medium are preferably the following components: 20 mg/L to 30 mg/L of CoCl₂.6H₂O, 100 mg/L to 200 mg/L of MnSO₄.4H₂O, 10 mg/L to 20 mg/L of CuCl₂.2H₂O, 20 mg/L to 40 mg/L of H₃BO₃, 20 mg/L to 30 mg/L of Na₂MoO₄.2H₂O, 100 mg/L to 150 mg/L of Zn(CH₃COO)₂.2H₂O, and 500 mg/L to 1,500 mg/L of Fe(III) citrate. More preferably, the trace elements are the following components: 25 mg/L of CoCl₂.6H₂O, 150 mg/L of MnSO₄.4H₂O, 15 mg/L of CuCl₂.2H₂O, 30 mg/L of H₃BO₃, 25 mg/L of Na₂MoO₄.2H₂O, 130 mg/L of Zn(CH₃COO)₂.2H₂O, and 1,000 mg/L of Fe(III) citrate. It should be understood that the trace elements in the fermentation tank medium of the present disclosure play the following two roles: 1. The trace elements serve as components constituting cells of the strain. 2. The trace elements serve as components for active groups of the enzyme or can maintain an activity of the enzyme. The addition of the trace elements in the present disclosure, especially the addition of iron (other trace elements play the role of serving as prosthetic groups or activators), greatly improves the activity and stability of myo-inositol oxygenase produced by the recombinant engineered strain, and provides a prominent foundation for the subsequent biological fermentation and conversion.

Further, with reference to the above embodiment, the present disclosure has no special restrictions on a type of the defoaming agent, and preferably, a commercially-available liquid defoaming agent is adopted. In the present disclosure, the defoaming agent is added at an amount of preferably 0.05 mL/L to 0.15 mL/L and more preferably 0.1 mL/L. It should be understood that the defoaming agent in the present disclosure can be added selectively according to a specific situation. For example, if there is too much foam on a liquid surface, the defoaming agent can be added manually according to a situation. The amount of the defoaming agent added should not be too large, and it is preferable to retain a small amount of foam. Otherwise, it is easy to cause the insufficient oxygen supply.

Further, with reference to the above embodiment, the present disclosure further includes: after the fermentation reaction is conducted for a specified period of time, a feeding medium is added to a reaction system at a specified feeding rate for feeding control. Preferably, the feeding control is conducted at preferably 10 h to 14 h and more preferably 12 h after the starting of the fermentation reaction. The feeding medium preferably includes: glucose: 500 g/L to 700 g/L, magnesium sulfate: 1 g/L to 3 g/L, a nitrogen-containing compound: 8 g/L to 12 g/L, and trace elements: 1,000 mg/L to 1,500 mg/L. The trace elements in the feeding medium of the present disclosure are preferably the following components: 20 mg/L to 30 mg/L of CoCl₂.6H₂O, 100 mg/L to 200 mg/L of MnSO₄.4H₂O, 10 mg/L to 20 mg/L of CuCl₂.2H₂O, 20 mg/L to 40 mg/L of H₃BO₃, 20 mg/L to 30 mg/L of Na₂MoO₄.2H₂O, 100 mg/L to 150 mg/L of Zn(CH₃COO)₂.2H₂O, and 500 mg/L to 1,500 mg/L of Fe(III) citrate. The nitrogen-containing compound in the feeding medium of the present disclosure is preferably selected from the group consisting of a peptone, a yeast powder, and a dry corn steep liquor powder and is more preferably a yeast powder. It should be understood that the feeding medium in the present disclosure is different from the above-mentioned fermentation tank medium mainly in a high glucose concentration. This is because a main function of the feeding medium is to supplement nutrients and necessary trace elements for strain cells in a system. In particular, in the present disclosure, the addition of the nitrogen-containing compound greatly improves the stability of myo-inositol oxygenase produced by strain cells and promotes the biological conversion of inositol.

Further, with reference to the above embodiment, in the present disclosure, during a fermentation reaction process, an inducing agent is added for induction control. In the present disclosure, the inducing agent is added preferably when an OD₆₀₀ value reaches 70 to 80 and more preferably when OD₆₀₀ is 75. In the present disclosure, the inducing agent is preferably a monosaccharide and more preferably arabinose. It should be understood that an effect of the inducing agent added in the present disclosure is to initiate the expression of a protein gene.

Further, with reference to the above embodiment, in the present disclosure, the feeding control is conducted in stages. Specifically, from 0 h to 3 h after starting of feeding, a feeding rate is controlled at preferably 600 g/h to 700 g/h and more preferably 650 g/h, from 3 h after the starting of the feeding to the addition of the inducing agent, the feeding rate is controlled at preferably 900 g/h to 1,100 g/h and more preferably 1,000 g/h, and after the addition of the inducing agent, the feeding rate is controlled at preferably 700 g/h to 800 g/h and more preferably 750 g/h. However, it has been verified by experiments that, when the feeding rate is controlled at 650 g/h from 0 h to 3 h after the starting of the feeding, the feeding rate is controlled at 1,000 g/h from 3 h after the starting of the feeding to the addition of the inducing agent, and the feeding rate is controlled at 750 g/h after the addition of the inducing agent, the technical effect of stable myo-inositol oxygenase production with a high yield can be achieved. It should be understood that the feeding control generally starts when the nutritional deficiency is resulted from the continuous expression of strain cells and the consumption of a medium to cause a significant decrease in a yield of the product enzyme. The feeding control conducted for the reaction system in this case can timely supplement nutrients for the strain cells to ensure the efficient output of myo-inositol oxygenase. In combination with the improvement of the trace elements in the fermentation tank medium for an activity of the enzyme, the feeding control enables the overall improvement of the activity, stability, and concentration of myo-inositol oxygenase, and provides a prominent foundation for the conversion of inositol.

In the present disclosure, after the fermentation broth is produced after the fermentation cultivation, the fermentation broth is centrifuged to obtain the wet strain cell. In the present disclosure, the centrifugation is conducted preferably by a centrifuge, the centrifugation is conducted at a rotational speed of preferably 14,000 rpm to 18,000 rpm and more preferably 16,000 rpm, and the centrifugation is conducted for preferably 15 min to 25 min and more preferably 20 min.

In another embodiment, in the present disclosure, the fermentation broth may also filtered with a membrane. The present disclosure has no special restrictions on the membrane, as long as the corresponding filtration and retention function can be implemented. The membrane is preferably a ceramic membrane. A pore size of the membrane is controlled at preferably 50 nm to 100 nm and more preferably 80 nm.

In the present disclosure, after the wet strain cell is obtained, the wet strain cell is added to a reaction solution for conversion to produce the GlcUA. The reaction solution is an inositol-containing reaction solution. Preferably, the reaction solution includes inositol with a mass fraction of 4 wt% to 7 wt% and boric acid with a concentration of 40 mM to 60 mM. More preferably, the reaction solution includes inositol with a mass fraction of 5 wt% and boric acid with a concentration of 50 mM.

In another embodiment, the reaction solution preferably includes inositol with a mass fraction of 4 wt% to 7 wt%, a phosphate with a concentration of 20 mM to 40 mM, and Fe²⁺ with a concentration of 2 mM to 4 mM, and more preferably includes inositol with a mass fraction of 5 wt%, a phosphate with a concentration of 30 mM, and Fe²⁺ with a concentration of 3 mM. The phosphate is preferably monopotassium phosphate.

Further, with reference to the above embodiment, in the present disclosure, the wet strain cell is added at an amount of preferably 25 g/L to 35 g/L and more preferably 30 g/L.

Further, with reference to the above embodiment, in the present disclosure, after the wet strain cell is added to the reaction solution, the conversion is conducted. During the conversion, a pH is adjusted to preferably 7 to 9 and more preferably 8, and a dissolved oxygen content is controlled at preferably 40% or more and more preferably 50%. The conversion is conducted for preferably 6 h to 8 h and more preferably 7 h.

Further, with reference to the above embodiment, after the conversion is conducted for a specified period of time, the present disclosure further includes: a specified amount of inositol is continuously added to a reaction system. In the present disclosure, the inositol is added at preferably 1.5 h to 2 h and more preferably 2 h after starting of the conversion. In the present disclosure, the inositol added is preferably inositol with a mass fraction of preferably 4 wt% to 6 wt% and more preferably 4.5 wt%. The inositol is preferably in a form of an aqueous solution. The inositol aqueous solution has a mass concentration of preferably 12 wt% to 15 wt% and more preferably 13 wt%. The inositol aqueous solution is added preferably in a fed-batch manner. The inositol aqueous solution is added for preferably 1.5 h to 2.5 h and more preferably 2 h.

Further, with reference to the above embodiment, after the conversion is completed, the present disclosure further includes: a conversion solution is filtered to collect a strain cell for recycling. In the present disclosure, the filtering is conducted preferably by a ceramic membrane, and a pore size of the ceramic membrane is preferably 50 nm to 100 nm and more preferably 80 nm. In the present disclosure, the recycling means that, after the wet strain cell is obtained through the filtering, the wet strain cell can be added to an inositol reaction solution to directly participate in the conversion of inositol.

In order to well illustrate the technical solution for the preparation of GlcUA in the present disclosure, the present disclosure also provides the following specific examples. It should be understood that the raw materials used in the following examples are commercially-available raw materials unless otherwise specified.

### Example 1

In this example, a preparation method of GlcUA was provided, including the following steps:
S1. Single colonies of a recombinant engineered strain were inoculated into 300 mL of an LB medium and subjected to shaking cultivation at 37°C and 220 rpm for 5.5 h to produce a primary seed culture.
S2. When OD₆₀₀ was 2.2, the primary seed culture was inoculated into 30 L of a seed tank medium at an inoculum size of 2%, and subjected to secondary seed cultivation until OD₆₀₀ was 2.12 to produce a seed culture.

A composition of the 30 L of the seed tank medium was as follows:
glucose: 1 wt%, monopotassium phosphate: 1 wt%, magnesium sulfate: 0.05 wt%, citric acid: 0.15 wt%, ammonium sulfate: 0.5 wt%, CoCl₂.6H₂O: 20 mg/L, MnSO₄.4H₂O: 120 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1 g/L, a defoaming agent: 0.1 ml/L, and water: the balance.

Conditions for the secondary seed cultivation were as follows:
a temperature: 37°C; an air flow rate: 0.5 m³/h; a rotational speed: 300 rpm; a pressure: 0.02 MPa; a pH: adjusting with ammonia water to a pH of 7; and a dissolved oxygen content: 20%.

S3. The seed culture was inoculated into 100 L of a fermentation tank medium at an inoculum size of 10%, and fermentation cultivation was conducted for 36 h to produce a fermentation broth.

A composition of the 100 L of the fermentation tank medium was as follows:
glucose: 1 wt%, monopotassium phosphate: 1 wt%, magnesium sulfate: 0.06 wt%, citric acid: 0.2 wt%, ammonium sulfate: 0.5 wt%, CoCl₂.6H₂O: 20 mg/L, MnSO₄.4H₂O: 120 mg/L, CuCl₂.2H₂O: 12 mg/L, H₃BO₃: 25 mg/L, Na₂MoO₄.2H₂O: 25 mg/L, Zn(CH₃COO)₂.2H₂O: 130 mg/L, Fe(III) citrate: 0.8 g/L, a defoaming agent: 0.1 ml/L, and water: the balance.

Conditions for the fermentation cultivation were as follows:
a temperature: 37°C; an air flow rate: 1.5 m³/h; a rotational speed: 200 rpm; a pressure: 0.02 MPa; a pH: adjusting with ammonia water to a pH of 7; and a dissolved oxygen content: 25%.

S4. A feeding medium was added to a reaction system in the S3 for feeding control. When OD₆₀₀ was 75, arabinose (an inducing agent) was added for induction.

Based on a mass of the feeding medium per liter, a composition of the feeding medium was as follows:
glucose: 600 g/L, magnesium sulfate: 2 g/L, a yeast powder: 10 g/L, CoCl₂.6H₂O: 25 mg/L, MnSO₄.4H₂O: 150 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1,000 mg/L, and water: the balance.

When a dissolved oxygen content increased significantly and a pH increased at about 12 h of cultivation in a cultivation tank, feeding was started. A feeding rate for the feeding control was as follows: the feeding rate was controlled at 700 g/h from 0 h to 3 h after starting of feeding, the feeding rate was controlled at 900 g/h from 3 h after the starting of the feeding to the addition of the inducing agent, and the feeding rate was controlled at 800 g/h after the addition of the inducing agent. The fermentation broth produced had OD₆₀₀ of 150.3.

S5. The fermentation broth was centrifuged at 18,000 rpm for 25 min to obtain a wet strain cell.

S6. The wet strain cell was added at 25 g/L to a reaction solution including inositol with a mass fraction of 5 wt% and boric acid with a concentration of 40 mM.

Conversion was conducted for 6 h at a pH of 8 and a dissolved oxygen content of 40% to produce the GlcUA.

Through the above steps, a molar conversion rate of inositol was 98.4% or more, a product had a GlcUA content of 53.0 g/L, and myo-inositol oxygenase could be recycled.

### Example 2

In this example, a preparation method of GlcUA was provided, including the following steps:
S1. Single colonies of a recombinant engineered strain were inoculated into 300 mL of an LB medium and subjected to shaking cultivation at 37°C and 200 rpm for 5.5 h to produce a primary seed culture.
S2. When OD₆₀₀ was 2.26, the primary seed culture was inoculated into 30 L of a seed tank medium at an inoculum size of 2%, and subjected to secondary seed cultivation until OD₆₀₀ was 2.12 to produce a seed culture.

A composition of the 30 L of the seed tank medium was as follows:
glucose: 2 wt%, monopotassium phosphate: 2 wt%, magnesium sulfate: 0.05 wt%, citric acid: 0.15 wt%, ammonium sulfate: 0.5 wt%, CoCl₂.6H₂O: 20 mg/L, MnSO₄.4H₂O: 120 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 0.8 g/L, a defoaming agent: 0.1 ml/L, and water: the balance.

Conditions for the secondary seed cultivation were as follows:
a temperature: 37°C; an air flow rate: 0.5 m³/h; a rotational speed: 300 rpm; a pressure: 0.02 MPa; a pH: adjusting with ammonia water to a pH of 7; and a dissolved oxygen content: 20%.

S3. The seed culture was inoculated into 100 L of a fermentation tank medium at an inoculum size of 10%, and fermentation cultivation was conducted for 36 h to produce a fermentation broth.

A composition of the 100 L of the fermentation tank medium was as follows:
glucose: 1 wt%, monopotassium phosphate: 2 wt%, magnesium sulfate: 0.08 wt%, citric acid: 0.1 wt%, ammonium sulfate: 0.5 wt%, CoCl₂.6H₂O: 20 mg/L, MnSO₄.4H₂O: 120 mg/L, CuCl₂.2H₂O: 12 mg/L, H₃BO₃: 25 mg/L, Na₂MoO₄.2H₂O: 25 mg/L, Zn(CH₃COO)₂.2H₂O: 130 mg/L, Fe(III) citrate: 0.8 g/L, a defoaming agent: 0.08 ml/L, and water: the balance.

Conditions for the fermentation cultivation were as follows:
a temperature: 37°C; an air flow rate: 1.5 m³/h; a rotational speed: 200 rpm; a pressure: 0.02 MPa; a pH: adjusting with ammonia water to a pH of 7; and a dissolved oxygen content: 25%.

S4. A feeding medium was added to a reaction system in the S3 for feeding control. When OD₆₀₀ was 70, arabinose (an inducing agent) was added for induction.

A composition of the feeding medium was as follows:
glucose: 600 g/L, magnesium sulfate: 2 g/L, a yeast powder: 10 g/L, CoCl₂.6H₂O: 25 mg/L, MnSO₄.4H₂O: 150 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1,000 mg/L, and water: the balance.

When a dissolved oxygen content increased significantly and a pH increased at about 12 h of cultivation in a cultivation tank, feeding was started. A feeding rate for the feeding control was as follows: the feeding rate was controlled at 700 g/h from 0 h to 3 h after starting of feeding, the feeding rate was controlled at 900 g/h from 3 h after the starting of the feeding to the addition of the inducing agent, and the feeding rate was controlled at 700 g/h after the addition of the inducing agent. The fermentation broth produced had OD₆₀₀ of 151.4.

S5. The fermentation broth was filtered through a 50 nm ceramic membrane to obtain a wet strain cell.

S6. The wet strain cell was added at 25 g/L to a reaction solution including inositol with a mass fraction of 7 wt% and boric acid with a concentration of 50 mM.

Conversion was conducted for 6 h at a pH of 8 and a dissolved oxygen content of 40% to produce the GlcUA.

S7. After the conversion was conducted for 2 h, an inositol aqueous solution with a mass concentration of 15 wt% was added to a reaction system in a fed-batch manner for 2 h. A mass of inositol added was 5 wt% of a total mass of the reaction system.

S8. A conversion solution was filtered with an 80 nm ceramic membrane to collect a strain cell for recycling.

Through the above steps, a molar conversion rate of inositol was 85.7% or more, a product had a GlcUA content of 89.3 g/L, and myo-inositol oxygenase could be recycled.

### Example 3

S1. Single colonies of a recombinant engineered strain were inoculated into 300 mL of an LB medium and subjected to shaking cultivation at 37°C and 220 rpm for 5 h to produce a primary seed culture.

S2. When OD₆₀₀ was 2.18, the primary seed culture was inoculated into 30 L of a seed tank medium at an inoculum size of 2%, and subjected to secondary seed cultivation until OD₆₀₀ was 2.08 to produce a seed culture.

A composition of the 30 L of the seed tank medium was as follows:
glucose: 1 wt%, monopotassium phosphate: 1 wt%, magnesium sulfate: 0.05 wt%, citric acid: 0.15 wt%, ammonium sulfate: 0.5 wt%, CoCl₂.6H₂O: 20 mg/L, MnSO₄.4H₂O: 120 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1 g/L, a defoaming agent: 0.1 ml/L, and water: the balance.

Conditions for the secondary seed cultivation were as follows:
a temperature: 37°C; an air flow rate: 0.5 m³/h; a rotational speed: 300 rpm; a pressure: 0.02 MPa; a pH: adjusting with ammonia water to a pH of 7; and a dissolved oxygen content: 20%.

S3. The seed culture was inoculated into 100 L of a fermentation tank medium at an inoculum size of 10%, and fermentation cultivation was conducted for 36 h to produce a fermentation broth.

A composition of the 100 L of the fermentation tank medium was as follows:
glucose: 1 wt%, monopotassium phosphate: 2 wt%, magnesium sulfate: 0.08 wt%, citric acid: 0.1 wt%, ammonium sulfate: 0.5 wt%, CoCl₂.6H₂O: 20 mg/L, MnSO₄.4H₂O: 120 mg/L, CuCl₂.2H₂O: 12 mg/L, H₃BO₃: 25 mg/L, Na₂MoO₄.2H₂O: 25 mg/L, Zn(CH₃COO)₂.2H₂O: 130 mg/L, Fe(III) citrate: 0.8 g/L, a defoaming agent: 0.08 ml/L, and water: the balance.

Conditions for the fermentation cultivation were as follows:
a temperature: 37°C; an air flow rate: 1.8 m³/h; a rotational speed: 220 rpm; a pressure: 0.03 MPa; a pH: adjusting with ammonia water to a pH of 7; and a dissolved oxygen content: 25%.

S4. A feeding medium was added to a reaction system in the S3 for feeding control. When OD₆₀₀ was 80, arabinose (an inducing agent) was added for induction.

Based on a mass of the feeding medium per liter, a composition of the feeding medium was as follows:
glucose: 600 g/L, magnesium sulfate: 2 g/L, a yeast powder: 10 g/L, CoCl₂.6H₂O: 25 mg/L, MnSO₄.4H₂O: 150 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1,000 mg/L, and water: the balance.

When a dissolved oxygen content increased significantly and a pH increased at about 12 h of cultivation in a cultivation tank, feeding was started. A feeding rate for the feeding control was as follows: the feeding rate was controlled at 650 g/h from 0 h to 3 h after starting of feeding, the feeding rate was controlled at 1000 g/h from 3 h after the starting of the feeding to the addition of the inducing agent, and the feeding rate was controlled at 750 g/h after the addition of the inducing agent. The fermentation broth produced had OD₆₀₀ of 152.3.

S5. The fermentation broth was filtered through a 75 nm ceramic membrane to obtain a wet strain cell.

S6. The wet strain cell was added at 30 g/L to a reaction solution including inositol with a mass fraction of 5 wt%, a phosphate with a concentration of 30 mM, and Fe²⁺ with a concentration of 3 mM.

Conversion was conducted for 8 h at a pH of 8 and a dissolved oxygen content of 50% to produce the GlcUA.

S7. After the conversion was conducted for 1.8 h, an inositol aqueous solution with a mass concentration of 12 wt% was added to a reaction system in a fed-batch manner for 1.5 h. A mass of inositol added was 5 wt% of a total mass of the reaction system.

S8. A conversion solution was filtered with a 100 nm ceramic membrane to collect a strain cell for recycling.

Through the above steps, a molar conversion rate of inositol was 95.7% or more, a product had a GlcUA content of 83.2 g/L, and myo-inositol oxygenase could be recycled.

### Example 4

In this example, a preparation method of GlcUA was provided, including the following steps:
S1. Single colonies of a recombinant engineered strain were inoculated into 300 mL of an LB medium and subjected to shaking cultivation at 35°C and 220 rpm for 5.5 h to produce a primary seed culture.
S2. When OD₆₀₀ was 2, the primary seed culture was inoculated into 30 L of a seed tank medium at an inoculum size of 2%, and subjected to secondary seed cultivation until OD₆₀₀ was 2.4 to produce a seed culture.

A composition of the 30 L of the seed tank medium was as follows:
glucose: 2 wt%, monopotassium phosphate: 1 wt%, magnesium sulfate: 0.05 wt%, citric acid: 0.15 wt%, ammonium sulfate: 0.6 wt%, CoCl₂.6H₂O: 20 mg/L, MnSO₄.4H₂O: 120 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 0.8 g/L, a defoaming agent: 0.1 ml/L, and water: the balance.

Conditions for the secondary seed cultivation were as follows:
a temperature: 37°C; an air flow rate: 0.5 m³/h; a rotational speed: 280 rpm; a pressure: 0.015 MPa; a pH: adjusting with ammonia water to a pH of 7; and a dissolved oxygen content: 30%.

S3. The seed culture was inoculated into 100 L of a fermentation tank medium at an inoculum size of 10%, and fermentation cultivation was conducted for 38 h to produce a fermentation broth.

A composition of the 100 L of the fermentation tank medium was as follows:
glucose: 1 wt%, monopotassium phosphate: 1 wt%, magnesium sulfate: 0.06 wt%, citric acid: 0.2 wt%, ammonium sulfate: 0.5 wt%, CoCl₂.6H₂O: 20 mg/L, MnSO₄.4H₂O: 120 mg/L, CuCl₂.2H₂O: 12 mg/L, H₃BO₃: 25 mg/L, Na₂MoO₄.2H₂O: 25 mg/L, Zn(CH₃COO)₂.2H₂O: 130 mg/L, Fe(III) citrate: 0.8 g/L, a defoaming agent: 0.1 ml/L, and water: the balance.

Conditions for the fermentation cultivation were as follows:
a temperature: 37°C; an air flow rate: 1.5 m³/h; a rotational speed: 200 rpm; a pressure: 0.02 MPa; a pH: adjusting with ammonia water to a pH of 7; and a dissolved oxygen content: 25%.

S4. A feeding medium was added to a reaction system in the S3 for feeding control. When OD₆₀₀ was 75, arabinose (an inducing agent) was added for induction.

Based on a mass of the feeding medium per liter, a composition of the feeding medium was as follows:
glucose: 500 g/L, magnesium sulfate: 3 g/L, a yeast powder: 8 g/L, CoCl₂.6H₂O: 25 mg/L, MnSO₄.4H₂O: 150 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1,000 mg/L, and water: the balance.

When a dissolved oxygen content increased significantly and a pH increased at about 10 h of cultivation in a cultivation tank, feeding was started. A feeding rate for the feeding control was as follows: the feeding rate was controlled at 700 g/h from 0 h to 3 h after starting of feeding, the feeding rate was controlled at 1000 g/h from 3 h after the starting of the feeding to the addition of the inducing agent, and the feeding rate was controlled at 700 g/h after the addition of the inducing agent. The fermentation broth produced had OD₆₀₀ of 156.8.

S5. The fermentation broth was filtered through a 100 nm ceramic membrane to obtain a wet strain cell.

S6. The wet strain cell was added at 35 g/L to a reaction solution including inositol with a mass fraction of 4 wt%, a phosphate with a concentration of 20 mM, and Fe²⁺ with a concentration of 4 mM.

Conversion was conducted for 7 h at a pH of 7 and a dissolved oxygen content of 50% to produce the GlcUA.

S7. After the conversion was conducted for 2 h, an inositol aqueous solution with a mass concentration of 15 wt% was added to a reaction system in a fed-batch manner for 2 h. A mass of inositol added was 4 wt% of a total mass of the reaction system.

S8. A conversion solution was filtered with a 100 nm ceramic membrane to collect a strain cell for recycling.

Through the above steps, a molar conversion rate of inositol was 96.8% or more, a product had a GlcUA content of 86.5 g/L, and myo-inositol oxygenase could be recycled.

### Example 5

In this example, a preparation method of GlcUA was provided, including the following steps:

S1. Single colonies of a recombinant engineered strain were inoculated into 300 mL of an LB medium and subjected to shaking cultivation at 37°C and 240 rpm for 6 h to produce a primary seed culture.

S2. When OD₆₀₀ was 2, the primary seed culture was inoculated into 30 L of a seed tank medium at an inoculum size of 2%, and subjected to secondary seed cultivation until OD₆₀₀ was 2 to produce a seed culture.

A composition of the 30 L of the seed tank medium was as follows:
glucose: 1 wt%, monopotassium phosphate: 1 wt%, magnesium sulfate: 0.05 wt%, citric acid: 0.15 wt%, ammonium sulfate: 0.5 wt%, CoCl₂.6H₂O: 20 mg/L, MnSO₄.4H₂O: 120 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1 g/L, a defoaming agent: 0.1 ml/L, and water: the balance.

Conditions for the secondary seed cultivation were as follows:
a temperature: 37°C; an air flow rate: 0.5 m³/h; a rotational speed: 300 rpm; a pressure: 0.02 MPa; a pH: adjusting with ammonia water to a pH of 7; and a dissolved oxygen content: 20%.

S3. The seed culture was inoculated into 100 L of a fermentation tank medium at an inoculum size of 10%, and fermentation cultivation was conducted for 36 h to produce a fermentation broth.

A composition of the 100 L of the fermentation tank medium was as follows:
glucose: 1 wt%, monopotassium phosphate: 2 wt%, magnesium sulfate: 0.08 wt%, citric acid: 0.1 wt%, ammonium sulfate: 0.5 wt%, CoCl₂.6H₂O: 20 mg/L, MnSO₄.4H₂O: 120 mg/L, CuCl₂.2H₂O: 12 mg/L, H₃BO₃: 25 mg/L, Na₂MoO₄.2H₂O: 25 mg/L, Zn(CH₃COO)₂.2H₂O: 130 mg/L, Fe(III) citrate: 0.8 g/L, a defoaming agent: 0.08 ml/L, and water: the balance.

Conditions for the fermentation cultivation were as follows:
a temperature: 37°C; an air flow rate: 1.8 m³/h; a rotational speed: 220 rpm; a pressure: 0.03 MPa; a pH: adjusting with ammonia water to a pH of 7; and a dissolved oxygen content: 25%.

S4. A feeding medium was added to a reaction system in the S3 for feeding control. When OD₆₀₀ was 80, arabinose (an inducing agent) was added for induction.

Based on a mass of the feeding medium per liter, a composition of the feeding medium was as follows:
glucose: 700 g/L, magnesium sulfate: 1 g/L, a yeast powder: 12 g/L, CoCl₂.6H₂O: 25 mg/L, MnSO₄.4H₂O: 150 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1,000 mg/L, and water: the balance.

When a dissolved oxygen content increased significantly and a pH increased at about 14 h of cultivation in a cultivation tank, feeding was started. A feeding rate for the feeding control was as follows: the feeding rate was controlled at 700 g/h from 0 h to 3 h after starting of feeding, the feeding rate was controlled at 900 g/h from 3 h after the starting of the feeding to the addition of the inducing agent, and the feeding rate was controlled at 750 g/h after the addition of the inducing agent. The fermentation broth produced had OD₆₀₀ of 153.1.

S5. The fermentation broth was centrifuged at 16,000 rpm for 20 min to obtain a wet strain cell.

S6. The wet strain cell was added at 30 g/L to a reaction solution including inositol with a mass fraction of 7 wt% and boric acid with a concentration of 40 mM.

Conversion was conducted for 8 h at a pH of 7 and a dissolved oxygen content of 50% to produce the GlcUA.

S7. After the conversion was conducted for 2 h, an inositol aqueous solution with a mass concentration of 15 wt% was added to a reaction system in a fed-batch manner for 2 h. A mass of inositol added was 4.5 wt% of a total mass of the reaction system.

S8. A conversion solution was filtered with a 50 nm ceramic membrane to collect a strain cell for recycling.

Through the above steps, a molar conversion rate of inositol was 86.2% or more, a product had a GlcUA content of 85.1 g/L, and myo-inositol oxygenase could be recycled.

### Example 6

In this example, a preparation method of GlcUA was provided, including the following steps:

S1. Single colonies of a recombinant engineered strain were inoculated into 300 mL of an LB medium and subjected to shaking cultivation at 40°C and 240 rpm for 6 h to produce a primary seed culture.

S2. When OD₆₀₀ was 2, the primary seed culture was inoculated into 30 L of a seed tank medium at an inoculum size of 2%, and subjected to secondary seed cultivation until OD₆₀₀ was 2.14 to produce a seed culture.

A composition of the 30 L of the seed tank medium was as follows:
glucose: 2 wt%, monopotassium phosphate: 1 wt%, magnesium sulfate: 0.05 wt%, citric acid: 0.15 wt%, ammonium sulfate: 0.6 wt%, CoCl₂.6H₂O: 20 mg/L, MnSO₄.4H₂O: 120 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 0.8 g/L, a defoaming agent: 0.1 ml/L, and water: the balance.

Conditions for the secondary seed cultivation were as follows:
a temperature: 37°C; an air flow rate: 0.5 m³/h; a rotational speed: 320 rpm; a pressure: 0.03 MPa; a pH: adjusting with ammonia water to a pH of 7; and a dissolved oxygen content: 30%.

S3. The seed culture was inoculated into 100 L of a fermentation tank medium at an inoculum size of 10%, and fermentation cultivation was conducted for 40 h to produce a fermentation broth.

A composition of the 100 L of the fermentation tank medium was as follows:
glucose: 1 wt%, monopotassium phosphate: 1 wt%, magnesium sulfate: 0.06 wt%, citric acid: 0.2 wt%, ammonium sulfate: 0.5 wt%, CoCl₂.6H₂O: 20 mg/L, MnSO₄.4H₂O: 120 mg/L, CuCl₂.2H₂O: 12 mg/L, H₃BO₃: 25 mg/L, Na₂MoO₄.2H₂O: 25 mg/L, Zn(CH₃COO)₂.2H₂O: 130 mg/L, Fe(III) citrate: 0.8 g/L, a defoaming agent: 0.1 ml/L, and water: the balance.

Conditions for the fermentation cultivation were as follows:
a temperature: 37°C; an air flow rate: 1.5 m³/h; a rotational speed: 200 rpm; a pressure: 0.02 MPa; a pH: adjusting with ammonia water to a pH of 7; and a dissolved oxygen content: 25%.

S4. A feeding medium was added to a reaction system in the S3 for feeding control. When OD₆₀₀ was 75, arabinose (an inducing agent) was added for induction.

Based on a mass of the feeding medium per liter, a composition of the feeding medium was as follows:
glucose: 600 g/L, magnesium sulfate: 3 g/L, a yeast powder: 8 g/L, CoCl₂.6H₂O: 25 mg/L, MnSO₄.4H₂O: 150 mg/L, CuCl₂.2H₂O: 15 mg/L, H₃BO₃: 30 mg/L, Na₂MoO₄.2H₂O: 25 mg/L, Zn(CH₃COO)₂.2H₂O: 120 mg/L, Fe(III) citrate: 1,000 mg/L, and water: the balance.

When a dissolved oxygen content increased significantly and a pH increased at about 11.5 h of cultivation in a cultivation tank, feeding was started. A feeding rate for the feeding control was as follows: the feeding rate was controlled at 700 g/h from 0 h to 3 h after starting of feeding, the feeding rate was controlled at 1100 g/h from 3 h after the starting of the feeding to the addition of the inducing agent, and the feeding rate was controlled at 800 g/h after the addition of the inducing agent. The fermentation broth produced had OD₆₀₀ of 161.2.

S5. The fermentation broth was centrifuged at 16,000 rpm for 15 min to obtain a wet strain cell.

S6. The wet strain cell was added at 35 g/L to a reaction solution including inositol with a mass fraction of 4 wt% and boric acid with a concentration of 60 mM.

Conversion was conducted for 7 h at a pH of 8 and a dissolved oxygen content of 50% to produce the GlcUA.

S7. After the conversion was conducted for 1.5 h, an inositol aqueous solution with a mass concentration of 12 wt% was added to a reaction system in a fed-batch manner for 2 h. A mass of inositol added was 4.5 wt% of a total mass of the reaction system.

S8. A conversion solution was filtered with a 70 nm ceramic membrane to collect a strain cell for recycling.

Through the above steps, a molar conversion rate of inositol was 97.6% or more, a product had a GlcUA content of 85.4 g/L, and myo-inositol oxygenase could be recycled.

### Comparative Example 1

Comparative Example 1 was different from Example 1 merely in that the nitrogen-containing compound in the feeding medium was replaced with trace elements at an equal amount.

The final results were as follows: The fermentation broth obtained in the S4 had OD₆₀₀ of 122.3, a molar conversion rate of inositol was 56.8% or more, and a product had a GlcUA content of 54.7 g/L.

A speculated reason is as follows: The absence of the nitrogen-containing compound may lead to the instability of inositol oxides produced during fermentation, which causes the decline in an inositol conversion rate.

### Comparative Example 2

Comparative Example 2 was different from Example 2 merely in that the trace elements in the seed tank medium were replaced with glucose at an equal amount.

The final results were as follows: A molar conversion rate of inositol was 53.6% or more, and a product had a GlcUA content of 42.7 g/L.

A speculated reason is as follows: The absence of trace elements leads to the lack of components for strain cells and the slow growth of strain cells. In addition, the lack of key trace elements to support active groups of the enzyme causes the decline of an activity of the enzyme and ultimately leads to the reduction of an inositol conversion rate.

### Comparative Example 3

Comparative Example 3 was different from Example 2 merely in that the trace elements in the fermentation tank medium were replaced with glucose at an equal amount.

The final results were as follows: A molar conversion rate of inositol was 45.2% or more, and a product had a GlcUA content of 34.7 g/L.

Compared with Comparative Example 2, the molar conversion rate of inositol and the GlcUA content are further reduced. This is because there is a large number of strain cells during the fermentation and thus there is a large demand for trace elements.

### Comparative Example 4

Comparative Example 4 was different from Example 2 merely in that the trace elements in the feeding medium were replaced with a nitrogen-containing compound at an equal amount.

The final results were as follows: A molar conversion rate of inositol was 68.2% or more, and a product had a GlcUA content of 56.4 g/L.

Compared with Comparative Examples 2 and 3, the molar conversion rate of inositol and the GlcUA content decrease slightly. This is because the nitrogen-containing compound itself is an organic substance and includes a small amount of trace elements. This part of trace elements may play a role of supplementing trace elements and improve an activity of the enzyme to some extent.

### Comparative Example 5

Comparative Example 5 was different from Example 2 merely in that Fe(III) citrate among the trace elements in the seed tank medium, the fermentation tank medium, and the feeding medium was replaced with CoCl₂.6H₂O at an equal amount.

The final results were as follows: A molar conversion rate of inositol was 36.8% or more, and a product had a GlcUA content of 22.5 g/L.

A speculated reason is as follows: Iron among the trace elements plays a key role in the activation of myo-inositol oxygenase, and plays a more important role in an activity of the enzyme than other trace elements.

### Comparative Example 6

Comparative Example 6 was different from Example 2 merely in that the feeding control was not adopted during the fermentation.

The final results were as follows: A molar conversion rate of inositol was 0, and a product had a GlcUA content of 0.

A reason is as follows: Without the feeding control, even if the inducing agent is added subsequently, the expression of the protein cannot be induced, resulting in the failed conversion of inositol.

### Comparative Example 7

Comparative Example 7 was different from Example 3 merely in that the feeding rate of the feeding control during the fermentation process is no longer set according to different stages, but a constant feeding rate of 650 g/h is kept throughout the fermentation process.

The final results were as follows: A molar conversion rate of inositol was 87% or more, and a product had a GlcUA content of 67.2 g/L.

A speculated reason is as follows: A fermentation process in a reaction system is not smooth before and after the addition of the inducing agent. If a same feeding rate is adopted all the time, the requirement of remaining a smooth fermentation process cannot be met. The sudden acceleration of a reaction after the addition of the inducing agent will also cause the shortage of nutrients, which will lead to the decrease in a reaction rate and the reduction of a product.

In addition, after GlcUA is prepared through the above method, GlcUA can be further converted into a derivative of GlcUA, which is glucuronolactone and commonly known as glucurone. In the prior art, glucuronolactone is prepared mainly through an esterification reaction of GlcUA with a compound acid reagent composed of phosphoric acid and sulfuric acid. However, when the esterification reaction is conducted with the compound acid reagent, due to the presence of sulfuric acid, a large amount of heat will be released from a reaction system in which there is water or water is produced, resulting in a too-high temperature in a reaction process. In addition, sulfuric acid has strong oxidizability. As a result, the carbonization of a reaction product will be aggravated, resulting in a yield loss. Based on this, in order to avoid the generation of water in the reaction process, it is necessary to control the reaction system at a relatively-high concentration. Generally, a Baume scale of a GlcUA concentrated solution should reach 46 to 47, and at this concentration, the reaction system has almost no fluidity. In addition, the esterification reaction should be strictly controlled at a low temperature of no more than 35°C, and the temperature cannot be increased for concentration halfway. Thus, a long reaction time is required, and the total preparation time from the reaction to the crystallization will be greater than 60 h. Due to the two-high concentration of the reaction system and the poor fluidity of the reaction system, ethanol must be added during the crystallization. Ethanol, as a flammable and explosive chemical, will cause a specified potential safety hazard when used in the production. In addition, in a reaction product prepared with this reaction system, glucuronolactone crystal grains are fine and have a grain size generally of greater than 50 mesh, resulting in difficult filtration. The reaction system also requires strictly-controlled cooling and stirring, and the reaction system needs to be cooled to -8°C, which makes the whole preparation process cumbersome and leads to an extremely-low production efficiency.

In order to solve the above-mentioned technical problems, an embodiment of the present disclosure also provides a preparation process of glucuronolactone, including the following steps:

Concentrated phosphoric acid is added to a GlcUA solution, an esterification reaction is conducted at a specified temperature under stirring, and crystallization is allowed to produce a crude glucuronolactone product.

In the above technical solution, the concentrated phosphoric acid is adopted instead of the mixed acid of phosphoric acid and sulfuric acid in the prior art, and a phosphoric acid concentration and a reaction temperature are controlled to allow the esterification reaction of the GlcUA solution to produce the glucuronolactone. When the above technical solution is used to prepare glucuronolactone, due to the excellent fluidity of a reaction system and the stirring condition, the total time from the starting of the reaction to the completion of the crystallization is less than 6 h, which greatly shortens the reaction-crystallization time, reduces the production cycle, and improves the production efficiency. Moreover, in the esterification reaction system of the present disclosure, only concentrated phosphoric acid is adopted and a temperature is controllable, such that the prepared glucuronolactone has advantages such as large crystal grains, easy filtration, and high yield.

Further, with reference to the above embodiment, the embodiment of the present disclosure further limits a solid content of the GlcUA solution to 50 wt% to 70 wt%. According to testing, this solid content is equivalent to a Baume scale of 20 to 30. The GlcUA solution has a viscosity of about 8 mpa·s to 12 mpa·s, indicating excellent fluidity. For example, the solid content of the GlcUA solution can be 60 wt% to 70 wt%. Specifically, the solid content of the GlcUA solution may be 50 wt%, 55 wt%, 60 wt%, 65 wt%, or 70 wt%. With the above solid content, the GlcUA solution has an appropriate viscosity. During the esterification reaction of the GlcUA solution with the concentrated phosphoric acid, a rotational speed for the stirring can generally be controlled at 80 rpm to 120 rpm, such that a system has prominent fluidity. Under the reaction system and reaction conditions in the embodiment of the present disclosure, ethanol is not required, that is, the crystallization can be directly conducted to produce a glucuronolactone crystal. In addition, the product has a large grain size of 20 mesh to 30 mesh.

Further, with reference to the above embodiment, the embodiment of the present disclosure further limits a mass concentration of the concentrated phosphoric acid to 70 wt% to 85 wt%. For example, the mass concentration of the concentrated phosphoric acid can be 80 wt% to 85 wt%. Specifically, the mass concentration of the concentrated phosphoric acid can be 70 wt%, 75 wt%, 80 wt%, or 85 wt%. The inventors have found through experimental verification that, only when the mass concentration of the concentrated phosphoric acid is limited in a specified range, the esterification reaction of the concentrated phosphoric acid with the GlcUA solution can proceed smoothly. When the mass concentration of the concentrated phosphoric acid is 70 wt% to 85 wt%, the solid content of the GlcUA solution is 50 wt% to 70 wt%, and the reaction temperature is 40°C to 80°C, a maximum esterification reaction rate can be achieved. In this case, there is a maximum yield of glucuronolactone, a glucuronolactone content in the crude glucuronolactone product can reach 95 wt% or more, and crystal grains are uniform and have a large grain size, which facilitates the filtration.

Further, with reference to the above embodiment, the embodiment of the present disclosure further limits an amount of the concentrated phosphoric acid to be 10 wt% to 50 wt% of a mass of a solid in the GlcUA solution. The esterification reaction is conducted for 1 h to 2 h.

The above technical solution of limiting the amount of the concentrated phosphoric acid to be 10 wt% to 50 wt% of the mass of the solid in the GlcUA solution can further shorten the time of the esterification reaction, improve the reaction efficiency, and increase the yield of the product. Under the above reaction conditions, the esterification reaction can be completed generally within 1 h to 2 h, which greatly shortens the reaction cycle and improves the reaction efficiency.

Further, with reference to the above embodiment, the embodiment of the present disclosure further limits the following content: After the esterification reaction and before the crystallization, the preparation process further includes: a product produced after the esterification reaction is subjected to water evaporation at 40°C to 80°C under a vacuum degree of less than or equal to -0.09 MPa, where a volume of evaporated water is 30% to 50% of a volume of a reaction solution.

Further, the crystallization includes: a product produced after the esterification reaction is subjected to dynamic gradient cooling crystallization at a rate of 5°C/h to 10°C/h with a crystallization termination temperature of 5°C to 15°C to obtain the crude glucuronolactone product.

When the above technical solution is adopted, during the whole esterification and crystallization process, due to the prominent fluidity of a reaction system, ethanol is not required, and by controlling the dynamic gradient cooling of the product produced after the esterification reaction at a rate of 5°C/h to 10°C/h, a glucuronolactone crystal can be directly crystallized with a large crystal grain size of 20 mesh to 30 mesh. A system produced after the crystallization is completed is a solid-liquid mixed system, which facilitates the separation of glucuronolactone through filtration. The crystallization process is simple. A total time of the esterification reaction and the crystallization can be minimized to 6 h. Therefore, the present disclosure overcomes the problems that there is a long crystallization time during a double-acid reaction process and a potential safety hazard is caused by the addition of ethanol during a crystallization process in the prior art. When the above technical solution is adopted, a glucuronolactone content in the crude glucuronolactone product is higher than or equal to 95 wt%, and a crystallization ratio is higher than or equal to 80%.

Further, the GlcUA solution in the embodiment of the present disclosure can be prepared from commercially-available GlcUA, or can be prepared through a reaction of an inositol solution with myo-inositol oxygenase, or can be prepared by any of the preparation methods in Examples 1 to 6 of the present disclosure. For example, when the GlcUA solution is prepared through a reaction of an inositol solution with myo-inositol oxygenase, the following steps are specifically included: The inositol solution is pre-prepared, and the myo-inositol oxygenase is added to the inositol solution for conversion to produce a conversion solution. For example, a conversion reaction between inositol and the myo-inositol oxygenase can conducted at a temperature of 30°C to 40°C and a pH of 8.0 to 9.0. The conversion solution is filtered and concentrated successively to obtain a GlcUA concentrated solution with a solid content of 50 wt% to 70 wt%. Further, it is tested by the inventors that the above solid content of the GlcUA concentrated solution is equivalent to a Baume scale of 20 to 30, and the GlcUA concentrated solution has a viscosity of 9 mpa.s to 10 mpa.s. Due to the use of the concentrated phosphoric acid-based esterification reaction system, the GlcUA concentrated solution only needs to have a concentration equivalent to the Baume scale of 20 to 30. The GlcUA concentrated solution with this concentration can be easily acquired with low concentration energy consumption. There is no need to replenish an acid during the crystallization process, and the crystallization process consumes a short time, which further shortens the reaction time.

Further, with reference to the above embodiment, the filtration in the embodiment of the present disclosure includes: The conversion solution is filtered through a ceramic membrane, and a ceramic-membrane filtrate is collected. A pore size of the ceramic membrane is 20 nm to 100 nm. The ceramic-membrane filtrate is filtered through an ultrafiltration membrane, and an ultrafiltration filtrate is collected. A pore size of the ultrafiltration membrane is 5,000 Da to 20,000 Da. The use of the ceramic membrane filtration and the ultrafiltration membrane filtration can achieve the purification of a GlcUA product, and can filter impurities out relatively rapidly, which has advantages such as high separation efficiency and excellent impurity removal effect.

Further, with reference to the above embodiment, in the embodiment of the present disclosure, after the filtering and before the concentrating for the conversion solution, the preparation process further includes: a filtrate is desalinated with a cation exchange resin to produce a desalinated solution with an electrical conductivity of less than 7,000 us/cm. For example, the cation exchange resin can be a strongly-acidic cation exchange resin. It should be understood that, unlike the preparation of a GlcUA solution from commercially-available GlcUA, during the conversion of inositol to prepare a GlcUA solution, a specified amount of a salt will be introduced, and the presence of the salt may affect the subsequent crystallization process. Therefore, the desalination is adopted, and the electrical conductivity of the desalinated solution is controlled. The lower the electrical conductivity, the easier it is to achieve crystallization. It has been verified through experiments that, when the electrical conductivity of the desalinated solution is controlled at less than 7,000 us/cm, crystals with large uniform grain sizes can be produced.

Further, with reference to the above embodiment, in the embodiment of the present disclosure, after the filtering and before the concentrating for the conversion solution, the preparation process further includes: a filtrate is subjected to adsorption with a macroporous adsorption resin for decolorization, and a decolorized solution is collected. For example, the macroporous adsorption resin can be LS-108 or LS-109D. A GlcUA solution produced after the reaction of inositol with myo-inositol oxygenase can be further decolorized to further improve a purity of the GlcUA solution, which facilitates the subsequent esterification reaction.

Further, the concentrating includes: A liquid to be concentrated is concentrated with a nanofiltration membrane, and a nanofiltration concentrate is collected. A solid content in the nanofiltration concentrate is 10 wt% to 15 wt% and a pore size of the nanofiltration membrane is 150 Da to 300 Da. The nanofiltration concentrate is concentrated in a concentrator to obtain the GlcUA concentrated solution with the solid content of 50 wt% to 70 wt%.

In the above technical solution in the embodiment of the present disclosure, before the esterification reaction, the GlcUA solution is subjected to two-stage dehydration, which reduces or avoids the dissolution loss of glucuronolactone in water to improve a crystallization yield of glucuronolactone.

In order to well illustrate the technical solution of the present disclosure, the present disclosure also provides the following specific examples to further illustrate the method for preparing glucuronolactone with GlcUA. It should be understood that the raw materials used in the following examples are commercially-available raw materials unless otherwise specified, and GlcUA can also be prepared through the above-mentioned preparation method as long as the performance requirements such as solid content and viscosity of GlcUA can be met after a treatment.

The operating parameters of the ceramic membrane, the nanofiltration membrane, and the ultrafiltration membrane adopted in the following examples are listed below as reference:

| Name | Temperature | Membrane-inlet pressure | Membrane-outlet pressure | Flux |
|---|---|---|---|---|
| Ceramic membrane | ≤35.0°C | 0.5-0.6MPa | 0.2-0.4MPa | 80L/h |
| Nanofiltration membrane | ≤35.0°C | 2.5-3.0MPa | 2.0-2.5MPa | 40L/h |
| Ultrafiltration membrane | ≤35.0°C | 0.3-0.7MPa | 0.2-0.6MPa | 150L/h |

### Example 7

In this example, a preparation process of glucuronolactone was provided, including the following steps:

S1. Concentrated phosphoric acid with a mass concentration of 70 wt% was added to 5.8 L of a GlcUA solution with a solid content of 73 wt% (viscosity: 12.4 mpa·s), and an esterification reaction was conducted for 1 h under stirring at a temperature of 80°C and a rotational speed of 120 rpm. An amount of the concentrated phosphoric acid added was 58% of a mass of a solid in the GlcUA solution.

S2. A product produced after the esterification reaction was subjected to dynamic gradient cooling crystallization at a rate of 5°C/h with a crystallization termination temperature of 5°C to produce a crude glucuronolactone product. The crude glucuronolactone product was subjected to suction filtration to produce a solid, and the solid was washed with 2.0 L of absolute ethanol and then vacuum-dried to obtain 3,161 g of a white crystal.

According to liquid chromatography testing, a glucuronolactone content in the white crystal was 95.3 wt%, a crystallization ratio was 82.3%, and a grain size of glucuronolactone was 20 mesh to 25 mesh.

### Example 8

In this example, a preparation process of glucuronolactone was provided, including the following steps:

S1. Concentrated phosphoric acid with a mass concentration of 72 wt% was added to 6.4 L of a GlcUA solution with a solid content of 50 wt% (viscosity: 8.1 mpa·s), and an esterification reaction was conducted for 2 h under stirring at a temperature of 40°C and a rotational speed of 80 rpm. An amount of the concentrated phosphoric acid added was 22% of a mass of a solid in the GlcUA solution.

S2. A product produced after the esterification reaction was subjected to water evaporation at 80°C under a vacuum degree of -0.09 MPa. A volume of evaporated water was 50% of a volume of a reaction solution.

S3. A system produced after the treatment in the S2 was subjected to dynamic gradient cooling crystallization at a rate of 10°C/h with a crystallization termination temperature of 10°C to produce a crude glucuronolactone product. The crude glucuronolactone product was subjected to suction filtration to produce a solid, and the solid was washed with 2.0 L of absolute ethanol and then vacuum-dried to obtain 2,453 g of a white crystal.

According to liquid chromatography testing, a glucuronolactone content in the white crystal was 95.7 wt%, a crystallization ratio was 84.5%, and a grain size of glucuronolactone was 25 mesh to 30 mesh.

### Example 9

In this example, a preparation process of glucuronolactone was provided, including the following steps:

S1. Concentrated phosphoric acid with a mass concentration of 84 wt% was added to 6.0 L of a GlcUA solution with a solid content of 70 wt% (viscosity: 11.8 mpa·s), and an esterification reaction was conducted for 1.5 h under stirring at a temperature of 78°C and a rotational speed of 120 rpm. An amount of the concentrated phosphoric acid added was 48% of a mass of a solid in the GlcUA solution.

S2. A product produced after the esterification reaction was subjected to water evaporation at 40°C under a vacuum degree of -0.09 MPa. A volume of evaporated water was 30% of a volume of a reaction solution.

S3. A system produced after the treatment in the S2 was subjected to dynamic gradient cooling crystallization at a rate of 5°C/h with a crystallization termination temperature of 10°C to produce a crude glucuronolactone product. The crude glucuronolactone product was subjected to suction filtration to produce a solid, and the solid was washed with 2.0 L of absolute ethanol and then vacuum-dried to obtain 3,361 g of a white crystal.

According to liquid chromatography testing, a glucuronolactone content in the white crystal was 97.7 wt%, a crystallization ratio was 88.2%, and a grain size of glucuronolactone was 20 mesh to 25 mesh.

### Example 10

In this example, a preparation process of glucuronolactone was provided, including the following steps:

S1. An inositol solution was pre-prepared, and myo-inositol oxygenase was added to the inositol solution for conversion to produce a conversion solution. Specific steps were as follows: A functional protein, L-cysteine, inositol, and ferrous sulfate were added to a buffer system to produce a reaction system. A ratio of the functional protein, the L-cysteine, the inositol, and the ferrous sulfate was 1 × 10⁵ µg : 2 mmol : 20 mmol : 1 mmol. An initial concentration of each component in the reaction system was as follows: the functional protein: 100 µg/mL, the L-cysteine: 2 mmol/L, the inositol: 0.3 mol/L, and Fe²⁺: 1 mmol/L. The buffer system was a 50 mM Tris-HCl buffer with a pH of 8.0. A conversion reaction was conducted for 6 h to 8 h at a temperature of 37°C and a pH of 8.0.

47.3 L of the conversion solution with a solid content of 5.6 wt% was filtered through a ceramic membrane with a pore size of 20 nm under the following conditions: an operating temperature: 35°C, a membrane-inlet pressure: 0.6 MPa, a membrane-outlet pressure: 0.4 MPa, and a membrane flux: 80 L/h, and a ceramic-membrane filtrate was collected. The ceramic-membrane filtrate was then filtered through an ultrafiltration membrane with a pore size of 5,000 Da under the following conditions: an operating temperature: 35°C, a membrane-inlet pressure: 0.7 MPa, a membrane-outlet pressure: 0.6 MPa, and a membrane flux: 150 L/h, and an ultrafiltration filtrate was collected. The ultrafiltration filtrate was desalinated with a strongly-acidic cation exchange resin to produce a desalinated solution with an electrical conductivity of 6,900 us/cm. The desalinated solution was further subjected to adsorption with an LS-109D macroporous adsorption resin for decolorization, and a decolorized solution was collected. The decolorized solution was concentrated by a nanofiltration membrane with a pore size of 150 Da under the following conditions: an operating temperature: 35°C, a membrane-inlet pressure: 3.0 MPa, a membrane-outlet pressure: 2.5 MPa, and a membrane flux: 40 L/h, and a nanofiltration concentrate was collected. A solid content in the nanofiltration concentrate was 10 wt%. The nanofiltration concentrate was concentrated in a concentrator to produce 3.9 L of a GlcUA concentrated solution with a solid content of 62 wt% (viscosity: 10.5 mpa·s).

S2. Concentrated phosphoric acid with a mass concentration of 76 wt% was added to 3.9 L of the GlcUA concentrated solution with the solid content of 62 wt%, and an esterification reaction was conducted for 1.5 h under stirring at a temperature of 60°C and a rotational speed of 100 rpm. An amount of the concentrated phosphoric acid added was 30 wt% of a mass of a solid in the GlcUA concentrated solution.

S3. A product produced after the esterification reaction was subjected to water evaporation at 70°C under a vacuum degree of -0.09 MPa. A volume of evaporated water was 35% of a volume of a reaction solution.

S4. A system produced after the treatment in the S3 was subjected to dynamic gradient cooling crystallization at a rate of 8°C/h with a crystallization termination temperature of 10°C to produce a crude glucuronolactone product. The crude glucuronolactone product was subjected to suction filtration to produce a solid, and the solid was washed with 2.0 L of absolute ethanol and then vacuum-dried to obtain 1,895 g of a white crystal.

According to liquid chromatography testing, a glucuronolactone content in the white crystal was 98.6 wt%, a crystallization ratio was 86.4%, and a grain size of glucuronolactone was 25 mesh to 30 mesh.

### Example 11

In this example, a preparation process of glucuronolactone was provided, including the following steps:

S1. An inositol solution was pre-prepared, and myo-inositol oxygenase was added to the inositol solution for conversion to produce a conversion solution. Specific steps were as follows: A functional protein, L-cysteine, inositol, and ferrous sulfate were added to a buffer system to produce a reaction system. A ratio of the functional protein, the L-cysteine, the inositol, and the ferrous sulfate was 1 × 10⁵ µg : 2 mmol : 20 mmol : 1 mmol. An initial concentration of each component in the reaction system was as follows: the functional protein: 100 µg/mL, the L-cysteine: 2 mmol/L, the inositol: 0.3 mol/L, and Fe²⁺: 1 mmol/L. The buffer system was a 50 mM Tris-HCl buffer with a pH of 8.0. A conversion reaction was conducted for 30 min at a temperature of 37°C and a pH of 8.0.

33.9 L of the conversion solution with a solid content of 6.1 wt% was filtered through a ceramic membrane with a pore size of 100 nm under the following conditions: an operating temperature: 35°C, a membrane-inlet pressure: 0.5 MPa, a membrane-outlet pressure: 0.2 MPa, and a membrane flux: 80 L/h, and a ceramic-membrane filtrate was collected. The ceramic-membrane filtrate was then filtered through an ultrafiltration membrane with a pore size of 20,000 Da under the following conditions: an operating temperature: 35°C, a membrane-inlet pressure: 0.3 MPa, a membrane-outlet pressure: 0.2 MPa, and a membrane flux: 150 L/h, and an ultrafiltration filtrate was collected. The ultrafiltration filtrate was desalinated with a strongly-acidic cation exchange resin to produce a desalinated solution with an electrical conductivity of 5,000 us/cm. The desalinated solution was further subjected to adsorption with an LS-108 macroporous adsorption resin for decolorization, and a decolorized solution was collected. The decolorized solution was concentrated by a nanofiltration membrane with a pore size of 300 Da under the following conditions: an operating temperature: 35°C, a membrane-inlet pressure: 2.5 MPa, a membrane-outlet pressure: 2.0 MPa, and a membrane flux: 40 L/h, and a nanofiltration concentrate was collected. A solid content in the nanofiltration concentrate was 15 wt%. The nanofiltration concentrate was concentrated in a concentrator to produce 3.4 L of a GlcUA concentrated solution with a solid content of 53 wt% (viscosity: 8.3 mpa·s).

S2. Concentrated phosphoric acid with a mass concentration of 74 wt% was added to 3.4 L of the GlcUA concentrated solution with the solid content of 53 wt%, and an esterification reaction was conducted for 2 h under stirring at a temperature of 45°C and a rotational speed of 85 rpm. An amount of the concentrated phosphoric acid added was 11% of a mass of a solid in the GlcUA concentrated solution.

S2. A product produced after the esterification reaction was subjected to water evaporation at 60°C under a vacuum degree of -0.09 MPa. A volume of evaporated water was 45% of a volume of a reaction solution.

S3. A system produced after the treatment in the S2 was subjected to dynamic gradient cooling crystallization at a rate of 10°C/h with a crystallization termination temperature of 10°C to produce a crude glucuronolactone product. The crude glucuronolactone product was subjected to suction filtration to produce a solid, and the solid was washed with 2.0 L of absolute ethanol and then vacuum-dried to obtain 1,401 g of a white crystal.

According to liquid chromatography testing, a glucuronolactone content in the white crystal was 96.1 wt%, a crystallization ratio was 85.7%, and a grain size of glucuronolactone was 20 mesh to 25 mesh.

### Example 12

In this example, a preparation process of glucuronolactone was provided, including the following steps:

S1. An inositol solution was pre-prepared, and myo-inositol oxygenase was added to the inositol solution for conversion to produce a conversion solution. Specific steps were as follows: A functional protein, L-cysteine, inositol, and ferrous sulfate were added to a buffer system to produce a reaction system. A ratio of the functional protein, the L-cysteine, the inositol, and the ferrous sulfate was 1 × 10⁵ µg : 2 mmol : 20 mmol : 1 mmol. An initial concentration of each component in the reaction system was as follows: the functional protein: 100 µg/mL, the L-cysteine: 2 mmol/L, the inositol: 0.3 mol/L, and Fe²⁺: 1 mmol/L. The buffer system was a 50 mM Tris-HCl buffer with a pH of 8.0. A conversion reaction was conducted for 30 min at a temperature of 37°C and a pH of 8.0.

42.2 L of the conversion solution with a solid content of 6.5 wt% was taken and vacuum-concentrated at a temperature of 70°C and a vacuum degree of -0.09 MPa to produce 3.9 L of a GlcUA concentrated solution with a solid content of 62 wt% (viscosity: 11.4 mpa·s).

S2. 0.48 L of concentrated phosphoric acid with a mass concentration of 79 wt% was added to 3.9 L of the GlcUA concentrated solution with the solid content of 62 wt%, and an esterification reaction was conducted for 1.5 h under stirring at a temperature of 70°C and a rotational speed of 110 rpm.

S3. A product produced after the esterification reaction was subjected to water evaporation at 70°C under a vacuum degree of -0.09 MPa. A volume of evaporated water was 39% of a volume of a reaction solution.

S4. A system produced after the treatment in the S3 was subjected to dynamic gradient cooling crystallization at a rate of 10°C/h with a crystallization termination temperature of 10°C to produce a crude glucuronolactone product. The crude glucuronolactone product was subjected to suction filtration to produce a solid, and the solid was washed with 2.0 L of absolute ethanol and then vacuum-dried to obtain 1,968 g of a white crystal.

According to liquid chromatography testing, a glucuronolactone content in the white crystal was 98.1 wt%, a crystallization ratio was 89.7%, and a grain size of glucuronolactone was 20 mesh to 25 mesh.

### Comparative Example 8

This comparative example was different from Example 7 merely in that the concentrated phosphoric acid with a mass concentration of 70 wt% was replaced by concentrated sulfuric acid with a mass concentration of 70 wt%.

According to testing, the crystalline glucuronolactone could not be produced with the reaction system in this comparative example, and the system would directly become a black viscous liquid.

### Comparative Example 9

This comparative example was different from Example 7 merely in that the concentrated phosphoric acid with a mass concentration of 70 wt% was replaced by a mixed acid of concentrated sulfuric acid with a mass concentration of 70 wt% and concentrated phosphoric acid with a mass concentration of 70 wt%. A mass ratio of the concentrated sulfuric acid to the concentrated phosphoric acid in the mixed acid was 1:1.

This reaction system would turn black, and a small amount of a gray crystal could be produced. According to liquid chromatography testing, a glucuronolactone content in the gray crystal was 62.3 wt%, a crystallization ratio was 21.4%, and a grain size of glucuronolactone was larger than 50 mesh.

It is speculated that a high moisture content in the reaction system may lead to the heat release of sulfuric acid during a reaction process, resulting in the increase of a carbonization ratio of a product.

### Comparative Example 10

This comparative example was different from Example 7 merely in that the esterification reaction was conducted at 35°C.

According to liquid chromatography testing, a glucuronolactone content in a product prepared under the above condition was 65.3 wt%, a crystallization ratio was 30.4%, and the product was powdery and had a grain size of larger than 50 mesh.

### Comparative Example 11

This comparative example was different from Example 7 merely in that the concentrated phosphoric acid with a mass concentration of 70 wt% was replaced by acetic acid.

According to testing, glucuronolactone was almost not produced under the above condition.

### Comparative Example 12

This comparative example was different from Example 10 merely in that the electrical conductivity of the desalinated solution was 7,500 us/cm.

According to liquid chromatography testing, a glucuronolactone content in a product prepared under the above condition was 85.1 wt%, a crystallization ratio was 51.3%, and the product was powdery and had a grain size of larger than 50 mesh.

### Test Example

Further, conditions for the liquid chromatography in Examples 7 to 12 and Comparative Examples 8 to 12 of the present disclosure were as follows:
a mobile phase: a 10 mmol/L formic acid aqueous solution;
a chromatographic column: a calcium column (300*7.7 or similar);
a flow rate: 0.5 mL/min;
a detector: a differential index detector;
a column temperature: 55°C;
a detector temperature: 45°C;
a solvent: a 10 mmol/L formic acid aqueous solution;
a standard concentration: 1.0 mg/mL (D-glucuronolactone [CAS No.] 32449-92-6, a content was 99.9% based on C6H8O6, the National Institutes for Food and Drug Control);
a test sample concentration: 1.0 mg/mL; and
chromatographic condition: running for 30 min with 100% of the 10 mmol/L formic acid aqueous solution.

A liquid chromatography spectrum of Example 10 was shown in FIG. 1 as an example. It can be seen from FIG. 1 that a peak at 16.383 min is a glucuronolactone peak, a peak at 24 min is a system peak, and a glucuronolactone content is 98.6%.

According to the data for the above examples, comparative examples, and test example: Under the conditions of the examples of the present disclosure, the combination of a GlcUA solution with a specified concentration and concentrated phosphoric acid with a specified concentration can make a viscosity of a system reach 8 mpa·s to 12 mpa·s. In this case, through stirring, the GlcUA solution can react with the concentrated phosphoric acid without the addition of ethanol, and with a total time from the reaction to the crystallization not exceeding 6 h, a crude crystal with uniform grain sizes in a range of 20 mesh to 30 mesh can be produced. The present disclosure has advantages such as short crystallization time, high production efficiency, large crystal grains, easy filtration, and high product yield.

The above are only preferred examples of the present disclosure, and is not intended to limit the present disclosure. Although the present disclosure is described in detail with reference to the above examples, a person skilled in the art can still make modifications to the technical solutions described in the above examples or equivalent substitutions to some technical features in the technical solutions. Any modifications, equivalent substitutions, improvements, etc. made within the spirit and scope of the present disclosure should be included within the protection scope of the present disclosure.

## Claims

1. A preparation method of D-glucuronic acid (GlcUA), comprising the following steps:
(1) cultivating a recombinant engineered strain to produce a seed culture;
(2) inoculating the seed culture into a fermentation tank medium, and conducting fermentation cultivation to produce a fermentation broth;
(3) collecting a wet strain cell from the fermentation broth through centrifugation or membrane filtration; and
(4) adding the wet strain cell to a reaction solution for conversion to produce the GlcUA,
wherein the step (2) comprises: after the seed culture is inoculated into the fermentation tank medium for cultivation, conducting feeding control and induction control.

2. The preparation method of GlcUA according to claim 1, wherein in the step (1), a cultivation process of the seed culture comprises:
A. primary seed cultivation: inoculating the recombinant engineered strain into an LB medium, and cultivating for 5 h to 6 h at 200 rpm to 240 rpm and 34°C to 40°C;
B. secondary seed cultivation: when OD₆₀₀ is 2 to 3, inoculating a resulting culture into a seed tank medium, and cultivating until OD₆₀₀ reaches 2 to 3 to obtain the seed culture,
wherein a specific composition of the seed tank medium is as follows: glucose: 1 wt% to 2 wt%, monopotassium phosphate: 1 wt% to 2 wt%, magnesium sulfate: 0.05 wt% to 0.08 wt%, citric acid: 0.1 wt% to 0.2 wt%, ammonium sulfate: 0.4 wt% to 0.6 wt%, trace elements: 1,000 mg/L to 1,500 mg/L, a defoaming agent: 0.05 ml/L to 0.15 ml/L, and water: the balance; and
conditions for the secondary seed cultivation are as follows:
a temperature: 34°C to 40°C; an air flow rate: 0.4 m³/h to 0.6 m³/h; a rotational speed: 280 rpm to 320 rpm; a pressure: 0.01 MPa to 0.03 MPa; a pH: adjusting with ammonia water to a pH of 7.0+0.1; and a dissolved oxygen content: 20% to 30%.

3. The preparation method of GlcUA according to claim 1, wherein in the step (2), the fermentation cultivation is conducted for 36 h to 40 h until OD₆₀₀ remains stable to obtain the fermentation broth;
wherein a specific composition of the fermentation tank medium is as follows: glucose: 1 wt% to 2 wt%, monopotassium phosphate: 1 wt% to 2 wt%, magnesium sulfate: 0.05 wt% to 0.08 wt%, citric acid: 0.1 wt% to 0.2 wt%, ammonium sulfate: 0.4 wt% to 0.6 wt%, trace elements: 1,000 mg/L to 1,500 mg/L, a defoaming agent: 0.05 ml/L to 0.15 ml/L, and water: the balance; and
conditions for the fermentation cultivation are as follows:
a temperature: 34°C to 40°C; an air flow rate: 1.2 m³/h to 1.8 m³/h; a rotational speed: 180 rpm to 220 rpm; a pressure: 0.01 MPa to 0.03 MPa; a pH: adjusting with ammonia water to a pH of 7.0+0.1; and a dissolved oxygen content: 20% to 30%.

4. The preparation method of GlcUA according to claim 1 or 3, wherein the feeding control is conducted as follows: after the fermentation cultivation is conducted for 10 h to 14 h, adding a feeding medium to a reaction system for the feeding control, wherein the feeding medium comprises: glucose: 500 g/L to 700 g/L, magnesium sulfate: 1 g/L to 3 g/L, a nitrogen-containing compound: 8 g/L to 12 g/L, trace elements: 1,000 mg/L to 1,500 mg/L, and water: the balance.

5. The preparation method of GlcUA according to claim 1 or 3, wherein the induction control is conducted as follows: adding an inducing agent when the OD₆₀₀ reaches 70 to 80.

6. The preparation method of GlcUA according to claim 5, wherein a feeding rate is controlled according to different stages, comprising: from 0 h to 3 h after starting of feeding, the feeding rate is controlled at 600 g/h to 700 g/h; from 3 h after the starting of the feeding to the addition of the inducing agent, the feeding rate is controlled at 900 g/h to 1,100 g/h; and after the addition of the inducing agent, the feeding rate is controlled at 700 g/h to 800 g/h.

7. The preparation method of GlcUA according to claim 1, wherein in the step (3), the centrifugation is conducted at 14,000 rpm to 18,000 rpm for 15 min to 25 min; and a filter membrane for the membrane filtration has a pore size of 50 nm to 100 nm.

8. The preparation method of GlcUA according to claim 1, wherein the reaction solution comprises inositol with a mass fraction of 4 wt% to 7 wt% and boric acid with a concentration of 40 mM to 60 mM; or
the reaction solution comprises the inositol with a mass fraction of 4 wt% to 7 wt%, a phosphate with a concentration of 20 mM to 40 mM, and Fe²⁺ with a concentration of 2 mM to 4 mM;
based on a total volume of the reaction solution, the wet strain cell is added at an amount of 25 g/L to 35 g/L; and
after the wet strain cell is added to the reaction solution, the preparation method further comprises: adjusting a pH to 7 to 9, and controlling a dissolved oxygen content at 40% or more; and the conversion is conducted for 6 h to 8 h.

9. The preparation method of GlcUA according to claim 8, wherein after the conversion is conducted for 1.5 h to 2 h, the preparation method further comprises: further adding inositol with a mass fraction of 4 wt% to 6 wt% to a reaction system, wherein the inositol is in a form of an aqueous solution with a mass concentration of 12 wt% to 15 wt%, and the inositol is added in a fed-batch manner for 1.5 h to 2.5 h.

10. The preparation method of GlcUA according to claim 1, wherein after the step (4), the preparation method further comprises: filtering a conversion solution to collect a strain cell for recycling.

11. A preparation process of glucuronolactone, comprising the following steps:
adding concentrated phosphoric acid to a GlcUA solution, conducting an esterification reaction at 40°C to 80°C under stirring, and allowing crystallization to produce a crude glucuronolactone product.

12. The preparation process of glucuronolactone according to claim 11, wherein a solid content of the GlcUA solution is 50 wt% to 70 wt%; a mass concentration of the concentrated phosphoric acid is 70 wt% to 85 wt%; an amount of the concentrated phosphoric acid added is 10 wt% to 50 wt% of a mass of a solid in the GlcUA solution; and the esterification reaction is conducted for 1 h to 2 h.

13. The preparation process of glucuronolactone according to claim 11, wherein after the esterification reaction and before the crystallization, the preparation process further comprises:
subjecting a product produced after the esterification reaction to water evaporation at 40°C to 80°C under a vacuum degree of less than or equal to -0.09 MPa, wherein a volume of evaporated water is 30% to 50% of a volume of a reaction solution.

14. The preparation process of glucuronolactone according to claim 11, wherein the crystallization comprises: subjecting a product produced after the esterification reaction to dynamic gradient cooling crystallization at a rate of 5°C/h to 10°C/h with a crystallization termination temperature of 5°C to 15°C to obtain the crude glucuronolactone product, wherein a glucuronolactone content in the crude glucuronolactone product is higher than or equal to 95 wt%, and a crystallization ratio is higher than or equal to 80%.

15. The preparation process of glucuronolactone according to claim 11, wherein the GlcUA solution is prepared through a reaction of an inositol solution with myo-inositol oxygenase.

16. The preparation process of glucuronolactone according to claim 15, wherein the reaction of the inositol solution with the myo-inositol oxygenase comprises the following steps:
pre-preparing the inositol solution, adding the myo-inositol oxygenase to the inositol solution for conversion to produce a conversion solution, and filtering and concentrating the conversion solution successively to obtain a GlcUA concentrated solution with a solid content of 50 wt% to 70 wt%.

17. The preparation process of glucuronolactone according to claim 16, wherein the filtering comprises:
filtering the conversion solution through a ceramic membrane, and collecting a ceramic-membrane filtrate, wherein a pore size of the ceramic membrane is 20 nm to 100 nm; and
filtering the ceramic-membrane filtrate through an ultrafiltration membrane, and collecting an ultrafiltration filtrate, wherein a pore size of the ultrafiltration membrane is 5,000 Da to 20,000 Da.

18. The preparation process of glucuronolactone according to claim 16, wherein after the filtering and before the concentrating for the conversion solution, the preparation process further comprises: desalinating a filtrate with a cation exchange resin to produce a desalinated solution with an electrical conductivity of less than 7,000 us/cm.

19. The preparation process of glucuronolactone according to claim 18, further comprising: subjecting the desalinated solution to adsorption with a macroporous adsorption resin for decolorization, and collecting a decolorized solution.

20. The preparation process of glucuronolactone according to claim 16, wherein the concentrating comprises:
concentrating a liquid to be concentrated with a nanofiltration membrane, and collecting a nanofiltration concentrate, wherein a solid content in the nanofiltration concentrate is 10 wt% to 15 wt% and a pore size of the nanofiltration membrane is 150 Da to 300 Da; and
concentrating the nanofiltration concentrate in a concentrator to obtain the GlcUA concentrated solution with the solid content of 50 wt% to 70 wt%.
